# EUROPÄISCHE PATENTSCHRIFT

(11) **EP 1 151 724 B1**
(45) Veröffentlichungstag und Bekanntmachung des Hinweises auf die Patenterteilung: **28.06.2006**
(21) Anmeldenummer: 01250150.8
(22) Anmeldetag: 27.04.2001
(51) Int. Cl.: A61B 17/32

(54) **Ultraschallzertrümmerer zum Zerkleinern oder Entfernen von Gewebe**
Ultrasound applicator for disintegration of tissue
Applicateur à ultrason pour la désintegration de tissus

(30) Priorität: 03.05.2000 DE 10021529
(43) Veröffentlichungstag der Anmeldung: 07.11.2001
(73) Patentinhaber: Celon AG Medical Instruments, 14513 Teltow (DE)
(72) Erfinder: Desinger, Kai, 12157 Berlin (DE)
(74) Vertreter: Eisenführ, Speiser & Partner

(56) Entgegenhaltungen:
- WO-A-95/17855
- WO-A-99/15120
- US-A- 5 123 903
- US-A- 5 167 619
- US-A- 5 728 130

## Beschreibung

Die Erfindung betrifft einen von Hand betätigbaren Ultraschallzertrümmerer zum Erzeugen von kleinen Kanälen in Muskelgewebe, bei denen sowohl über den Leistungsultraschall Druckamplituden/Stosswellen ins umgebende Gewebe eingebracht, als auch simultan ein definiert thermischer Eintrag ins umliegende Gewebe eingebracht werden kann. Ferner dient die Erfindung zum Zerkleinern oder Entfernen von menschlichem oder tierischem Gewebe, mit einer Sonotrode zum Übertragen von Ultraschallwellen und von lokal begrenzter Hochfrequenzspannung an ihrem distalen Ende an das Gewebe, einem an das proximale Ende der Sonotrode ankoppelbaren Ultraschallwandler und einem längs der Sonotrode verlaufenden Kanal zum Spülen und/oder Absaugen von zertrümmertem und/oder abgetragenem Gewebe und/oder ferner zur Übertragung einer hochfrequenten Wechselspannung in bipolarer Technik. Angeregt wird der Ultraschallwandler über einen externen Ultraschallgenerator, die Sonotrode mit ihrer in umittelbarer Nähe befindlichen Rückleitelektrode ferner mit HF-Spannung über eine externe Hochfrequenzspannungsquelle versorgt.Die Sonotrode dient hier zur simultanen Übertragung von Leistungsultrachall und Hochfrequenzspannung.

Ultraschallwellen hoher Leistung werden bekanntermaßen zum Zertrümmern und/oder Abtragen von Tumorgewebe eingesetzt. Die zu zerstörenden Tumorzellen platzen aufgrund der hohen Druckamplituden und Kavitationserscheinungen der eingesetzten Ultraschallwellen hoher Leistungsdichte. Ein herkömmlicher, von Hand betätigbarer Ultaschallzertrümmerer enthält einen Ultraschallwandler, in der Regel einen Piezo-Verbundwandler, der mittelbar oder unmittelbar an eine Sonotrode angekoppelt ist. Die Sonotrode dient dazu, die über ihre aktive Fläche von dem Ultraschallwandler erzeugten Ultraschallwellen an das abzutragende Gewebe in Form von Longitudinalwellen zu übertragen. Vorzugsweise wird bei den bekannten Geräten noch ein mechanischer Amplitudentransformator zwischen dem Ultraschallwandler und der Sonotrode geschaltet, der dafür sorgt, dass die an die Sonotrode übertragenen Ultraschallwellen eine geeignete Amplitude zum Zertrümmern und Abtragen der Tumorzellen aufweisen. Der Ultraschallwandler, der Amplitudentransformator und die Sonotrode befinden sich bei den bekannten Geräten in einem länglichen, griffartigen Gehäuse, wobei das distale Ende der Sonotrode aus einer distalen Öffnung des Gehäuses hervorragt, an dem das Gerät von Hand gehalten und geführt werden kann.

Bekannt ist aus der industriellen Anwendung von Leistungsultraschall ist das Ultraschallbohren von Werkstoffen. Durch die hohen mechanischen Abtragskräfte beim Leistungsultraschall, lassen sich bei geeigneten Sonotrodengeometrien in harten und weichen Materialien ohne wesentlichen Kraftaufwand Löcher oder Kanäle in verschiedensten Materialien erzeugen.

Unter dem Schlagwort "Trans-Myokardiale Laser-Revaskularisierung" werden derzeit überwiegend gepulste Lasersysteme eingesetzt, wobei insbesondere derzeit Geräte der Firmen PLC/USA, CARDIO GENESIS/USA und United State Surgical Coporation/USA zum Einsatz kommen. Mit diesen gepulsten Lasersystemen gelingt unter Ausnutzung des Mechanismus der sogenannten Photoablation das Anlegen von transmyokardialen Kanälen und systembedingt entstehen hierbei thermisch beeinflußte Randzonen und durch den Prozeß der Photoablation auch Stoßwellen. Allerdings lassen sich die Größe der thermisch beeinflußten Randzone und die Amplituden und Tiefenwirkung der Stoßwellen nicht getrennt voneinander regeln und optimieren. Außerdem sind diese Systeme extrem teuer.

Überraschenderweise konnte festgestellt werden, daß die bisher beim Einsatz dieser hochenergetischen Lasersysteme erzielten Erfolge, sich im wesentlichen auf zwei laserinduzierte Effekte zurückführen lassen:
Die Erzeugung intramuskulärer Stoßwellen durch den Prozeß der Photoablation einer schnellen lokalen thermischen Explosion zur Verdampfung des Gewebes in der Zielregion sowie durch die um Grundsatz bei dieser Art der Laseranwendung (gemeint ist der Prozeß der Photoablation) letzlich unvermeidbare thermische Schädigung der Randzonen, die je nach benutzten Einstellparametern der o. g. Laser von der Karbonisierung (d. h. Verkohlung) über Koagulation zur extremen Hyperthermie reicht. Auch für Experten auf diesem Gebiet völlig überraschend konnte nun gezeigt werden, daß die bisher berichteten akuten Erfolge dieses Verfahrens im wesentlichen auf sekundäre Effekte der entstehenden Stoßwellen und damit verbundenen Druckamplituden zurückzuführen sind und daß die Langzeiterfolge im wesentlichen auf die Ausbildung der thermisch beeinflußten Randzone der angelegten Kanäle zurückzuführen ist. Bei den nach dem Stand der Technik eingesetzten Systemen ist es a priori völlig unmöglich, die Wirkungsweise der Stoßwellen, d. h. die entstehende Druckamplitude und Druckstoßdauer sowie die damit verbundene Tiefenwirkung getrennt von denen bei Durchführung des Verfahrens auftretenden thermischen Randschädigungen zu optimieren. Ebenso ist es völlig unmöglich, die erkennbar vorteilhafte Ausbildung einer thermischen Randzone getrennt von den Stoßwellen zur Erzielung und Optimierung der berichteten Langzeiterfolge weiter zu optimieren.

Bei der allgemeinen Zertrümmerung von Gewebe lässt es sich trotz der Selektivität des Ultraschalls nicht verhindern, dass auch kleinere Gefäße zertrümmert werden. Die Folge sind Blutungen, die sich mit dem Ultraschallinstrument nicht stillen lassen, da es sich bei der Ultraschallzertrümmerung um einen athermischen Prozess handelt. Die Lösung dieses Problems wird bei einigen bekannten Geräten dadurch erreicht, dass eine bevorzugt aus Titan ausgebildete Hohlsonotrode mit einem hochfrequenten Wechselstrom (HF-Strom) gespeist wird. Die an dem distalen Ende des Gerätes in dem Gewebe erzeugten elektromagnetischen Hochfrequenzfelder koagulieren durch eine entsprechende Wärmeentwicklung das umliegende Gewebe, wodurch schließlich eine Blutung gestillt werden kann.

Bei dieser bekannten Erzeugung eines elektromagnetischen Hochfrequenzfeldes wird die Sonotrode als eine Elektrode geschaltet, die Gegenelektrode wird von außen auf den menschlichen Körper - im Bereich der Behandlungszone - aufgesetzt. Es handelt sich dabei um eine sogenannte monopolare Geräteanordnung, bei welcher der Strom durch einen Großteil des Körpers bis zu der Gegenelektrode fliesst, die in der Regel an einer der Extremitäten angebracht ist.

Aus dem Patent US 5,312,329 ist ein Gerät der eingangs genannten Art bekannt, bei dem ein rohrförmiger Hohlkanal entlang der Längsachse der Sonotrode vorgesehen ist. Die Mündung des Kanals wird von der aktiven Fläche der Sonotrode umgeben. Der Kanal führt in das Innere des Gehäuses und ist von den übrigen im Gehäuse befindlichen elektronischen und mechanischen Komponenten isoliert. Eine Vakuumpumpe kann an den Kanal angeschlossen werden, um Gewebefragmente oder Körperflüssigkeit oder den bei der Koagulation erzeugten Rauch durch ein in dem Hohlraum erzeugtes Vakuum aus dem Körper abzusaugen. Der Kanal wird ebenso dazu genutzt, eine Flüssigkeit zu dem Behandlungsgebiet der Sonotrode zuzuführen, um die Zellfragmente zu verflüssigen und daraufhin abzusaugen. Zu diesem Zweck lässt sich der Kanal mit einen Flüssigkeitsbehälter verbinden, der die Spülflüssigkeit bereitstellt.

Die aus der US 5 312 329 bekannte Anordnung von Sonotrode und Kanal hat zur Folge, dass die aktive Fläche der Sonotrode, d.h. die Stirnfläche der Sonotrode an ihrem distalen Ende, die für die Übertragung der Ultraschallwellen an das Gewebe sorgt, relativ klein ist, aber durch den internen Spülkanal der Außendurchmesser dennoch relativ gross ist. Weil die Abtragrate einer Sonotrode bei konstanter Frequenz und konstanter Amplitude in etwa proportional zu deren aktiven Fläche ist, bewirkt die Kanalmündung in der aktiven Fläche der Sonotrode eine Verringerung der aktiven Fläche und damit der Abtragrate. Dieser Verringerung wird dadurch abgeholfen, dass der Außendurchmesser der aktiven Ringfläche vergrößert wird. Dadurch wird bei diesem bekannten Gerät jedoch die Sicht auf das abzutragende Gewebe eingeschränkt, und das Einsatzgebiet des Ultraschallzertrümmerers wird dadurch auf relativ großflächige Strukturen eingeschränkt. Da im Haupteinsatzgebiet der Ultraschallzertrümmerer der Neurochirurgie jedoch immer kleinere, miniaturisierte Instrumente gefordert werden, um ein präzises Operieren zu ermöglichen, ist dieses bekannte Instrument nicht zufriedenstellend. Da der Durchmesser der Sonotroden bekannter Geräte aus den genannten Gründen bei einigen Millimetern liegt, sind die Sonotroden dieser bekannten Geräte auch starr und lassen sich nicht mehr flexibel einsetzen.

Aus WO95/17855 ist ein bipolarer Ultraschallzertrümmerer bekannt, der eine Sonotrode mit einer zentralen Bohrung zum Aufsaugen von abgetragenem Körpergewebe oder Blut aufweist.

Aus WO99/15120 ist ein Ultraschallzertrümmerer bekannt, der ausschließlich dazu ausgebildet ist, Ultraschallenergie in das Gewebe zu übertragen, jedoch keine elektrische Energie.

Stand der Technik sind außerdem die US-A-5,312,329, WO 99/04709 A1, US-A-4,931,047, US-A-5,776,092 und US-A-5,012,797.

Es ist daher eine Aufgabe der vorliegenden Erfindung, einen von Hand betätigbaren Ultraschallzertrümmerer der eingangs genannten Art bereitzustellen, der eine hohe Abtragrate bei einer aktiven Sonotrodenfläche mit kleinem Außendurchmesser erreicht, und der dabei auch eine effektive Spülung oder Absaugung von Zellfragmenten ermöglicht sowie die Möglichkeit eröffnet über einen lokalen hochfrequenten Stromfluss Blutungen thermisch zu stillen.

Eine weitere Aufgabe der Erfindung liegt darin, einen von Hand betätigbaren Ultraschallzertrümmerer der eingangs genannten Art bereitzustellen, der ein effizientes Ultraschallbohren von kleinen Kanälen in Muskelgewebe bei gleichzeitigem Eintrag von thermischer Energie in die Randzonen des Kanals mittels eines hochfrequenten elektromagnetischen Wechselfeldes ermöglicht, wobei der hochfrequente Stromfluss über eine bipolare Elektrodenanordnung in der Nähe des distalen Endes der Sonotrode realisiert wird.

Die Aufgabe der vorliegenden Erfindung wird durch einen von Hand betätigbaren Ultraschallzertrümmerer zum Entfernen von menschlichem oder tierischem Gewebe gemäß Anspruch 1 gelöst. Unter einer Vollfläche ist hier eine einfach zusammenhängende Fläche zu verstehen, d.h. eine Fläche, deren äußere Umrandung keine Aussparungen bzw. Löcher einschließt.

Die Vorteile der Erfindung liegen insbesondere darin, dass die aktive Fläche der Sonotrode, welche die Abtragrate beeinflusst, gleich der Vollfläche ist, dass also die Größe der von ihrer Außenkontur definierten aktiven Fläche nicht durch Hohlkanäle oder Bohrungen reduziert ist. Ferner kann durch den außerhalb der Sonotrode verlaufenden ersten Kanal das zertrümmerte Gewebe gespült oder abgesaugt werden, die Spül- und/oder Absaugfunktion ist also durch den außenliegenden ersten Kanal, und gegebenenfalls auch noch durch einen zweiten Kanal verwirklicht. Ferner können durch diesen Kanal/diese Kanäle auch Medikamente oder andere Substanzen an das Gewebe oder in den Gewebekanal eingebracht werden. Die Sonotrode ist in einem ersten Rohr angeordnet, dessen lichter Querschnitt größer als der Querschnitt der Sonotrode ist, so dass der Kanal zwischen dem ersten Rohr und der Sonotrode einen vorgegebenen Kanalquerschnitt besitzt. Die vorstehende Anordnung von Sonotrode und erstem Kanal ermöglicht einen kompakten Aufbau des distalen Endes des Ultraschallzertrümmerers. Die Kanalmündung umfasst die Sonotrode, weshalb zertrümmertes Gewebe in der Umgebung der Sonotrode effektiv abgesaugt werden kann. Im Bereich des distalen Endes einschließlich der Vollfläche bildet die Sonotrode eine erste Elektrodenoberfläche und ein die Sonotrode umgebendes Rohr weist eine zweite Elektrodenoberfläche auf. Dabei ist die Sonotrode selbst als Elektrode ausgebildet, das umgebende Absaugrohr kann als Elektrode ausgebildet sein. Die erste und auch die zweite Elektrodenoberfläche sind an eine äußere elektrische Spannungsquelle anschließbar. Erfindungsgemäß bilden daher die erste und die zweite Elektrodenoberfläche gemäß dieser Ausführungsform eine bipolare Elektrodenanordnung, welche bewirkt, dass über die Elektrodenoberflächen erzeugte Feldlinien vorzugsweise von der ersten Elektrodenoberfläche zu der zweiten Elektrodenoberfläche verlaufen. Die vorliegende bipolare Elektrodenanordnung sorgt also dafür, dass aufgrund des definierten Feldlinienverlaufs ein lokal genau eingegrenzter Gewebe-bereich in der Umgebung der ersten und zweiten Elektrodenoberflächen koaguliert wird und daher am Ort des durch Ultraschall erfolgenden Gewebeabtrages anschließend mit ein und derselben Gerätespitze dann über ein lokales hochfrequentes Feld und dem entsprechenden Stromfluß Blutungen gestillt werden können.

Eine vorteilhafte Ausgestaltung der vorliegenden Erfindung wird dadurch erzielt, dass die Vollfläche am distalen Ende der Sonotrode mit einer ebenen distalen Stirnfläche der Sonotrode zusammenfällt. Die distale Stirnfläche ist der Bereich, der bei der Applikation des Ultraschallzertrümmerers auf das Behandlungsgebiet aufgesetzt wird, und die Ultraschallwellen in das abzutragende oder zu bohrende Gewebe einleitet.

Nach einer weiteren vorteilhaften Ausgestaltung der Erfindung besitzt die Sonotrode einen uniformen Vollquerschnitt und ist als länglicher Stab ausgebildet. Als Material für die Sonotrode wird besonders bevorzugt Titan eingesetzt, es eignen sich jedoch auch andere körperverträgliche Materialien. Ein uniformer Querschnitt der Sonotrode ist vorteilhaft, da er sich besonders einfach herstellen lässt. Aufgrund eines oder mehrerer außen liegender Kanäle kann der Durchmesser der Sonotrode besonders klein gewählt werden, er liegt beispielsweise im Bereich von 0,3 bis 2 Millimetern. Aufgrund der geringen Größe des Sonotrodendurchmessers ist es auch möglich, die Sonotrode flexibel, biegsam zu gestalten, wodurch die Erreichbarkeit der gewünschten Behandlungsorte verbessert ist und das System auch über flexible Endoskope oder Katheter einsetzbar ist.

Nach einer weiteren Ausführungsform wird die Sonotrode zentral in dem ersten Rohr angeordnet. Die hat den Vorteil, dass der Bereich um das distale Ende der Sonotrode gleichförmig abgesaugt wird.

Die Sonotrode ragt bevorzugt aus der distalen Mündung des ersten Kanals axial hervor. Dies ist vorteilhaft, weil die Sicht auf die aktive Fläche der Sonotrode durch die vorliegenden Anordnung erleichtert wird und die Vorteile der Erfindung, maximal aktive Fläche bei kleinstmöglichem Durchmesser der Sonotrode voll zur Wirkung kommt. Durch den Unterdruck der Absaugung kann das zertrümmerte Gewebe dennoch effizient mit dem zurückgesetzten Absaugrohr entfernt werden.

Die Sonotrode und das erste Rohr sind gemäß einer weiteren Ausgestaltung der Erfindung axial relativ zueinander verschiebbar, um den Abstand zwischen der Ultraschallwellen übertragenden Stirnfläche der Sonotrode und der Rohrmündung einzustellen.

Der Ultraschallwandler wird bevorzugt über einen Amplitudentransformator an die Sonotrode angekoppelt. Der Amplitudentransformator sorgt dafür, dass die an die Sonotrode übertragenen Ultraschallwellen eine ausreichend große Amplitude erhalten, die geeignet ist, die zu entfernenden Zellen zu zertrümmern. Die Ultraschallamplitude kann über den von außen angeschlossenen Wechselstromgenerator, der den Ultraschallwandler erregt, vorteilhafterweise den jeweils vorliegenden Bedingungen geeignet angepasst werden.

Gemäß einer Weiterbildung der Erfindung wird ein proximaler Abschnitt der Sonotrode, der Amplitudentransformator und der Ultraschallwandler in einem stabförmigen Gehäuse untergebracht, aus dem ein distaler Abschnitt der Sonotrode herausragt, und der Kanal wird durch das Gehäuse hindurchgeführt. Das gemeinsame Gehäuse besitzt Anschlüsse für die elektrische Stromversorgung, für die Sauganschlüsse und/oder Spülanschlüsse zu den entsprechenden Pumpen. Das Gehäuse ist handgerecht geformt, so dass sich das Gerät leicht von Hand führen und einsetzen läßt.

Gemäß der erfindungsgemäßen Ausgestaltung der Erfindung besteht die Sonotrode und vorzugsweise auch das Kanalrohr aus einem elektrischen Leiter, der an die HF-Spannungsquelle anschließbar ist. Die Oberfläche der Sonotrode und des Rohrs bildet demnach die erste und zweite Elektrodenoberfläche. Dieser Ausführungsform ermöglicht einen unkomplizierten Aufbau des Ultraschallzertrümmerers, da die HF-Spannungsquelle direkt an das Rohr und die Sonotrode angeschlossen werden können. Es müssen zudem keine zusätzlichen Elektrodenoberflächen bereitgestellt werden.

Alternativ ist die Sonotrode aus einem elektrisch leitenden Material ausgebildet, das mindestens abschnittweise mit einer dielektrischen Isolatorschicht beschichtet ist. Das erste Rohr besteht bei dieser Ausführungsform aus einem leitenden Material, welches auf seiner Innenfläche eine dielektrische Isolatorschicht aufweisen kann, die besonders dann notwendig ist, wenn die Sonotrode keine solche Isolatorschicht trägt.

Gemäß einer weiteren Ausgestaltung der Erfindung besteht das Rohr aus einem elektrischen Isolator und die zweite Elektrodenfläche ist eine Metallbeschichtung auf dem Rohr. Vorteilhaft ist dabei, dass es möglich wird, durch die Größe und Form der Elektrodenoberflächen, das Koagulationsgebiet dem Einsatzgebiet des Ultraschallzertrümmerers entsprechend zu gestalten.

Gemäß einer besonders bevorzugten Ausführungsform der Erfindung ist außerhalb der Sonotrode ein zweiter Kanal vorgesehen, der ebenfalls längs der Sonotrode verläuft. Der erste Kanal und/oder der zweite Kanal lassen sich an einem vorgegebenen Ort am Umfang der Sonotrode anordnen, sie verlaufen parallel zur Sonotrode und anschließend durch das Gehäuse des Ultraschallzertrümmerers hindurch und lassen sich an äußere Pumpen (nicht dargestellt) anschließen, welche einen der Kanäle als Absaugkanal, den anderen als Spülkanal betreiben. Zur Verwirklichung des zweiten Kanals lässt sich auch an das erste Rohr konzentrisch ein zweites Rohr anordnen, welches den zweiten Kanal mit Ringquerschnitt bildet, der das erste Rohr umgibt. Das zweite Rohr endet bevorzugt eine vorgegebene Länge vor dem distalen Ende des ersten Rohres.

Zur Erzeugung von feinen Kanälen in Muskelgewebe zur transmyokardialen Revaskularisation (TMR) des Herzens, kann gemäß einer weiteren Ausgestaltung das Außenrohr, welches hier etwas flächiger oder balliger am distalen Ende ausgeführt ist, auf das Muskelgewebe aufgesetzt werden. Dieses die Sonotrode umgebende Instrumententeil kann sowohl rohrförmig ausgebildet die Sonotrode ganz umschliessen, oder aber auch zur Längsachse der Sonotrode schalenförmig ausgelegt, also offen sein. Durch ein definiertes Vorschieben der innenliegenden Sonotrode über einen Betätigungsmechansimus am Handstück und Aktivierung sowohl des Ultraschall- als auch des HF-Generators, können durch die von einem lokalen, hochfrequenten elektromagnetischen Wechselfeld HF-unterstützte Sonotrode feine Känale ins Muskelgewebe gebohrt werden. Durch den Ultraschall und das HF-Feld wird eine Druckamplitude als auch ein thermischer Eintrag in dem sich bildenen Kanal in dem Muskelgewebe erzeugt und damit eine Neubildung von Gefässen initiiert.

Vorteilhafte Ausführungsbeispiele der Erfindung sind in der Zeichnung darstellt und werden im folgenden näher beschrieben.

Es zeigen:
- Fig. 1: einen Ultraschallzertrümmerer gemäß einem ersten Ausführungsbeispiel; und
- Fig. 2: eine vergrößerte Darstellung der Sonotrode, des Saugkanals und des Spülkanals eines Ultraschallzertrümmerers gemäß einem zweiten Ausführungsbeispiel.
- Figur 3: eine vergrößerte Darstellung des distalen Endes der Sonotrode gemäß einem dritten Ausführungsbeispiel.

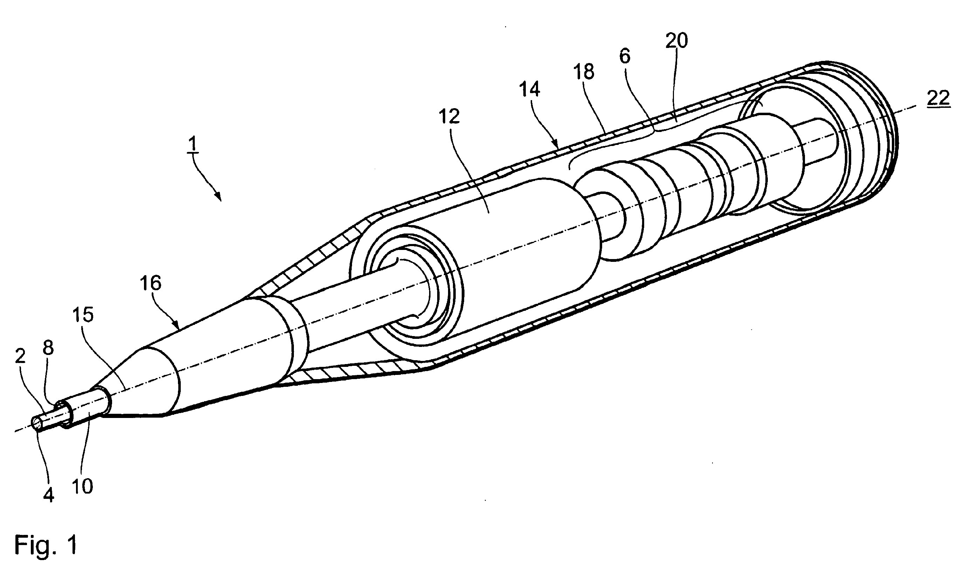

Fig. 1 zeigt ein erstes Ausführungsbeispiel eines von Hand betätigbaren Ultraschallzertrümmerers 1. Dargestellt ist ein im wesentlichen zylindrisches Gehäuse 14 mit einem kegelförmigen distalen Abschnitt 16. Die Gehäusewand 18 ist transparent dargestellt, so dass der Innenraum 20 des Gehäuses 14 sichtbar ist. Zur Längsachse 15 des Gehäuses 14 ist ein Ultraschallwandler 6 und ein Amplitudentransformator 12 angeordnet. Der distale Bereich des Amplitudentransformators 12 ist mit der Sonotrode 2 verbunden. Der Ultraschallwandler 6 befindet sich zwischen dem Amplitudentransformator 12 und dem proximalen Ende 22 des Gehäuses 14. Der Ultraschallwandler 6 ist an den Amplitudentransformator 12 angekoppelt und an dem Gehäuse 14 befestigt. Ein von außen an das Gerät anschließbarer elektrischer Wechselstromgenerator (nicht dargestellt) versorgt den Ultraschallwandler 6 mit der erforderlichen elektrischen Wechselspannung und Leistung, um den Ultraschallwandler 6 so anzuregen, dass dieser Ultraschallwellen abgibt. Von außen sind an das Gerät ferner noch Saug- und Spülpumpen (nicht dargestellt) sowie gegebenenfalls weitere elektrische Versorgungsquellen für Zusatzfunktionen anschließbar.

Der kegelförmige Abschnitt 16 des Gehäuses 14 geht distal in ein Rohr 10 über, dessen Längsachse mit der Längsachse 15 des Gehäuses 14 zusammenfällt. Aus der distalen Öffnung des Rohrs 10 ragt die Sonotrode 2 hervor. Die zylinderförmige Sonotrode 2 ist in dem lichten Querschnitt des Rohrs 10 zentral angeordnet. Die Stirnfläche 4 der Sonotrode 2 besitzt eine kreisförmige Umfangskontur; die Sonotrode 2 besitzt die Form eines schlanken Kreiszylinders, dessen Längsachse mit der Längsachse 15 des Gehäuses 14 fluchtet. Alternativ kann die Längsachse der Sonotrode 2 auch gegen die Längsachse 15 des Gehäuses 14 einen vorgegebenen Winkel einschließen. Der Vollquerschnitt des Kreiszylinders bildet am distalen Ende die aktive Fläche der Sonotrode 2. Die Sonotrode 2 besitzt bevorzugt eine Länge, die ein Vielfaches der halben Wellenlänge (λ/2) der angeregten Ultraschallwellen beträgt, um einen hohen Ultraschall-Leistungseintrag in das Gewebe zu ermöglichen. Die Sonotrode 2 besitzt einen geringen Querschnitt und ist bevorzugt aus biegbarem Material hergestellt. Das Rohr 10, welches die Sonotrode mit Abstand umgibt und einen Kanal 8 um die Elektrode herum bildet, ist am distalen Ende gegenüber der Sonotrode 2 verkürzt, so dass die distale Spitze des Gerätes, an dem der Gewebeabtrag erfolgt, nur von der Sonotrode gebildet ist.

Der Zwischenraum zwischen der Sonotrode 2 und dem Rohr 10 bildet den distalen Bereich eines Kanals 8, der durch das Gehäuse 14 verläuft. Der Kanal 8 wird zum Absaugen von abgetragenem oder zerstörtem Gewebe an eine Vakuumpumpe (nicht gezeigt) angeschlossen. Der Kanal 8 kann sowohl zum Absaugen als auch zum Spülen von Gewebe im Behandlungsgebiet eingesetzt werden. Der Kanal 8 kann zudem auch abwechselnd zum Spülen und Absaugen betrieben werden.

Die Sonotrode 2 besteht aus einem Vollmaterial, das zur Übertragung von Ultraschallwellen geeignet ist. Der ringförmige Hohlraum zwischen der Sonotrode 2 und dem Rohr 10 bildet die Mündung des Kanals 8. Die Sonotrode 2 lässt sich entlang ihrer Längsachse verschieben und ragt dann mehr oder weniger aus der Mündung des Kanals 8 hervor. Alternativ ist die Sonotrode unverschiebbar, verschiebbar ausgebildet ist dann das Rohr 10, um die Kanalmündung den Bedürfnissen des jeweiligen Behandlungsortes anzupassen. Die zu applizierenden Schallwellen werden über die Stirnfläche 4 der Sonotrode 2 in das zu behandelnde Gewebe übertragen.

Im vorliegenden Ausführungsbeispiel bildet die Sonotrode 2 eine erste Elektrode und das Rohr 10 eine zweite Elektrode. Die Sonotrode 2 und das Rohr 10 sind daher aus einem leitfähigen Material ausgebildet und werden an eine HF-Spannungsquelle (nicht gezeigt) angeschlossen. Da die Sonotrode 2 und das Rohr 10 relativ zueinander verschiebbar sind, kann mit einer derartigen Einstellung nicht nur die Kanalmündung, sondern auch die Form des elektromagnetischen Wechselfeldes zwischen den beiden Elektroden und damit das entsprechende Koagulationsgebiet geeignet eingestellt werden.

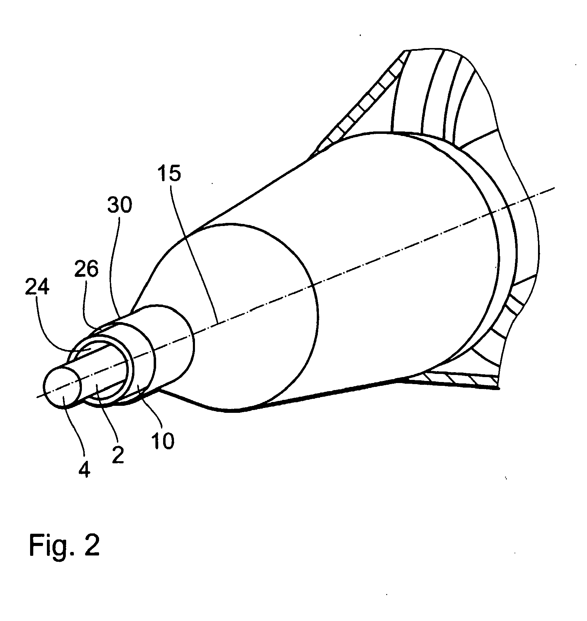

Fig. 2 zeigt eine vergrößerte Darstellung einer Sonotrode 2, die von zwei äußeren Kanälen, beispielsweise einem Saugkanal 24 und einem Spülkanal 26 umgeben ist. Die zylinderförmige Sonotrode 2 ragt wiederum aus der distalen Mündung eines Rohrs 10 hervor. Die Längsachse 3 der zylinderförmigen Sonotrode 2 liegt auf der Längsachse des Rohrs 10 und die kreisscheibenförmige Stirnfläche 4 der Sonotrode 2 bildet die aktive Fläche der Sonotrode 2 . Der Raum zwischen der Sonotrode 2 und dem Rohr 10 bildet die Mündung eines Saugkanals 24, d.h. desjenigen Kanals, der zum Absaugen von Zellfragementen eingesetzt wird. Der Saugkanal 24 verläuft durch das Gehäuse 14 hindurch zu einem proximalen Anschlussstutzen, an dem geeignete Saugeinrichtungen anschließbar sind. Ein weiteres Rohr 30 umschließt das Rohr 10 mit der Sonotrode 2 derart, dass ein distaler Längsabschnitt des Rohrs 10 und der Sonotrode 2 aus der Mündung des Rohrs 30 hervorragen.

Der Zwischenraum zwischen dem Rohr 30 und dem Rohr 10 bildet den distalen Abschnitt eines Spülkanals 26, d.h. eines Kanals, der zum Zuführen von Spülflüssigkeit in die Umgebung der Sonotrode 2 eingesetzt wird. Der Spülkanal 26 verläuft ebenfalls durch das Gehäuse 14.

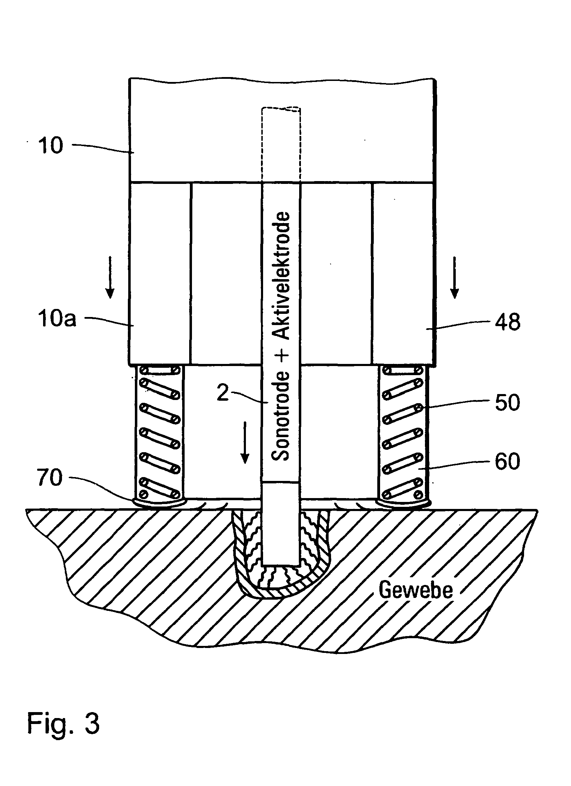

Figur 3 zeigt eine vergrößerte Darstellung der Sonotrode 2 einer weiteren Ausführungsform, wobei nur das distale Ende des Gerätes dargestellt ist. Die Sonotrode 2 ist bei dieser Ausführungsform wiederum zentral in dem Rohr 10 angeordnet. Die Wandung des Rohres 10 ist zu dem distalen Ende hin über einen vorgegebenen Umfangsabschnitt, beispielsweise 180°, weggebrochen und besitzt dadurch etwa einen halbkreisförmigen Querschnitt. An den axialen Kanten des verbleibenden Rohrquerschnitts 10a sind Führungen 48 vorgesehen, in denen Spiralfedern 50 lagern, welche mit ihrem distalen Ende gegen einen verschiebbaren distalen Wandabschnitt 60 anliegen. Der distale Wandabschnitt 60 des Rohres 10 ist - gegen die Vorspannung der Druckfedern 50 - in den axialen Führungen 48 des Rohres 10 verschiebbar geführt, so dass die Sonotrode 2 bei dem Ultraschall-Betrieb in das angrenzende Gewebe eindringen kann und dabei unter dem Andruck des Benutzers den verschiebbaren Wandungsabschnitt 60 des Rohres 10 axial in proximaler Richtung verschiebt.

Durch ein definiertes Vorschieben der innenliegenden Sonotrode 2 über einen Betätigungsmechanismus und bei gleichzeitiger Aktivierung sowohl des Ultraschall als auch des HF-Generators können durch die von dem elektromagnetischen Wechselfeld unterstützte Sonotrode feine Kanäle in das angrenzende Gewebe gebohrt werden. Durch den Energieeintrag mittels der Sonotrode und/oder einer bipolaren HF-Energiebeaufschlagung erfolgt auch ein thermischer Eintrag in die Kanalwand, der eine Neubildung von Gefäßen initiieren kann.

Um die in Figur 3 dargestellte Ausführungsform der Erfindung bipolar mit einem HF-Wechselspannungsfeld betreiben zu können, ist die Sonotrode 2 an einen äußeren HF-Generator angelegt, die entsprechende Gegenelektrode 70 ist an der Stirnkante der verschiebbaren distalen Rohrwandung 60 ausgebildet und stützt sich von außen auf das Gewebe auf, in dem die Sonotrode 2 einen dünnen Kanal erzeugen soll.

## Patentansprüche

1. Von Hand betätigbarer Ultraschallzertrümmerer zum Entfernen von menschlichem oder tierischem Gewebe, mit
einer Sonotrode (2) zum Übertragen von Ultraschallwellen an ihrem distalen Ende (4) in das Gewebe, wobei die Sonotrode (2) von einem ersten Rohr (10) umgeben ist, welches einen ersten Kanal (8) zwischen sich und der Sonotrode (2) bildet und die Sonotrode (2) aus einem elektrisch leitendem Material besteht, und das erste Rohr (10) eine zweite Elektrodenoberfläche aufweist,
einem über einen Amplitudentransformator (12) an die Sonotrode (2) ankoppelbaren Ultraschallwandler (6), und
einem längs der Sonotrode (2) verlaufenden ersten Kanal (8) zum Spülen oder Absaugen von zertrümmertem und/oder abgetragenem Gewebe,
**dadurch gekennzeichnet, dass** das distale Ende der Sonotrode (2) eine Vollfläche zum Übertragen der Ultraschallwellen an das Gewebe aufweist, die keine Aussparungen einschließt, und die Sonotrode im Bereich ihres distalen Endes einschließlich der Vollfläche eine erste Elektrodenoberfläche bildet und dass die erste und zweite Elektrodenoberfläche an eine äußere elektrische HF-Spannungsquelle anschließbar sind.

2. Ultraschallzertrümmerer nach Anspruch 1, **dadurch gekennzeichnet, dass** die Vollfläche am distalen Ende der Sonotrode (2) mit der distalen Stirnfläche (4) der Sonotrode (2) zusammenfällt.

3. Ultraschallzertrümmerer nach Anspruch 1 oder 2, **dadurch gekennzeichnet, dass** die Sonotrode (2) einen uniformen Vollquerschnitt besitzt und eine Länge besitzt, die im wesentlichen n x Lambda/2 beträgt, wobei Lambda die Wellenlänge der erzeugten Ultraschallwellen ist und n = 1, 2, 3... gilt.

4. Ultraschallzertrümmerer nach Anspruch 1, **dadurch gekennzeichnet, dass** die Sonotrode (2) zentral in dem ersten Rohr (10) angeordnet ist.

5. Ultraschallzertrümmerer nach einem der vorstehenden Ansprüche, **dadurch gekennzeichnet, dass** die Sonotrode (2) aus dem distalen Ende des ersten Rohres (10) axial einen vorgegebenen Längenabschnitt hervorragt.

6. Ultraschallzertrümmerer nach einem der vorstehenden Ansprüche, **dadurch gekennzeichnet, dass** die Sonotrode (2) und das erste Rohr (8) axial relativ zueinander verschiebbar sind.

7. Ultraschallzertrümmerer nach einem der vorstehenden Ansprüche, **gekennzeichnet durch** einen zweiten Kanal (26), der außerhalb und längs der Sonotrode (2) verläuft.

8. Ultraschallzertrümmerer nach einem der vorstehenden Ansprüche, **dadurch gekennzeichnet, dass** der erste Kanal (8; 24) und/oder der zweite Kanal (26) an einem vorgegebenen Ort am Umfang der Sonotrode (2) angeordnet ist und parallel zur Sonotrode (2) verläuft.

9. Ultraschallzertrümmerer nach Anspruch 7, **dadurch gekennzeichnet, dass** der zweite Kanal (26) an einem vorgegebenen Ort am Außenumfang des ersten Rohrs (10) angeordnet ist und parallel zur Sonotrode (2) verläuft.

10. Ultraschallzertrümmerer nach Anspruch 7, **dadurch gekennzeichnet, dass** konzentrisch um das erste Rohr (10) ein zweites Rohr (30) angeordnet ist, welches den zweiten Kanal (26) mit Ringquerschnitt bildet, der in Längsrichtung der Sonotrode (2) verläuft.

11. Ultraschallzertrümmerer nach einem der vorstehenden Ansprüche, **dadurch gekennzeichnet, dass** das proximale Ende der Sonotrode (2), der Amplitudentransformator (12) und der Ultraschallwandler (6) in einem Gehäuse (14) untergebracht sind, und dass der erste Kanal (8) durch das Gehäuse (14) hindurchgeführt ist.

12. Ultraschallzertrümmerer nach einem der vorstehenden Ansprüche, **dadurch gekennzeichnet, dass** das erste oder das zweite Rohr (10, 30) aus einem elektrischen Leiter bestehen, die an eine äußere elektrische HF-Spannungsquelle anschließbar sind.

13. Ultraschallzertrümmerer nach einem der vorstehenden Ansprüche 1 bis 11, **dadurch gekennzeichnet, dass** das erste und/oder das zweite Rohr (10) aus einem elektrischen Isolator besteht und die zweite Elektrodenoberfläche eine Metallbeschichtung auf dem ersten oder zweiten Rohr (10, 30) ist.

14. Ultraschallzertrümmerer nach einem der vorstehenden Ansprüche, **dadurch gekennzeichnet, dass** die Sonotrode (2) mindestens abschnittweise mit einer dielektrischen Isolatorschicht beschichtet ist.

15. Ultraschallzertrümmerer nach Anspruch 12, **dadurch gekennzeichnet, dass** das erste Rohr (10) und/oder das zweite Rohr (30) und an der Innenfläche mit einer dielektrischen Isolatorschicht versehen sind.

16. Ultraschallzertrümmerer nach einem der vorstehenden Ansprüche, **dadurch gekennzeichnet, dass** die Sonotrode (2) und das erste Rohr (10) sowie gegebenenfalls das zweite Rohr (30) aus einem elastischen Material bestehen.

17. Ultraschallzertrümmerer nach einem der vorstehenden Ansprüche, **dadurch gekennzeichnet, dass** die Sonotrode (2) und das erste Rohr (10) sowie gegebenenfalls das zweite Rohr (30) auswechselbar an dem Instrument befestigbar sind.

18. Ultraschallzertrümmerer nach einem der Ansprüche 1 bis 7, **dadurch gekennzeichnet, dass** die Wandung des ersten Rohrs (10) an dem distalen Rohrende teilweise weggebrochen ist.

19. Ultraschallzertrümmerer nach Anspruch 18, **dadurch gekennzeichnet, dass** aus der Wandung des ersten Rohres am distalen Rohrende ein Umfangsabschnitt weggebrochen ist.

20. Ultraschallzertrümmerer nach Anspruch 18 oder 19, **dadurch gekennzeichnet, dass** das erste axiale Rohr (10) oder die Sonotrode (2) axial unter Federvorspannung verschiebbar sind.

21. Ultraschallzertrümmerer nach Anspruch 20, **dadurch gekennzeichnet, dass** das distale Ende (60) des ersten Rohres (10), dessen Wandung abschnittsweise am Umfang weggebrochen ist, axial unter Federvorspannung gegen einen proximal angrenzenden Rohrabschnitt (10a) verschiebbar ist.

## Claims

1. Manually operable ultrasonic disintegrator for removing human or animal tissue, comprising a
sonotrode (2) for the transmission of ultrasonic waves at its distal end (4) into the tissue, whereby the sonotrode (2) is surrounded by a first tube (10) which forms a first channel (8) between itself and the sonotrode (2) and the sonotrode (2) is made from an electricity-conducting material, and the first tube (10) has a second electrode surface,
an ultrasonic transducer (6) which can be coupled to the sonotrode (2) via an amplitude transformer (12), and
a first channel (8) which extends along the sonotrode (2) for flushing and/or suctioning off disintegrated and/or ablated tissue,
**characterised in that** the distal end of the sonotrode (2) has a full surface for transmission of ultrasonic waves to the tissue, which does not include any recesses, and the sonotrode in the region of its distal end including the full surface forms a first electrode surface, and **in that** the first and second electrode surface can be connected to an external electrical HF voltage source.

2. Ultrasonic disintegrator according to claim 1, **characterised in that** the full surface at the distal end of the sonotrode (2) coincides with the distal end face (4) of the sonotrode (2).

3. Ultrasonic disintegrator according to claim 1 or 2, **characterised in that** the sonotrode (2) has a uniform full cross-section and has a length which is substantially n x lambda/2, where lambda is the wavelength of the ultrasonic waves produced and n = 1, 2, 3 ...

4. Ultrasonic disintegrator according to claim 1 **characterised in that** the sonotrode (2) is arranged centrally in the first tube (10).

5. Ultrasonic disintegrator according to one of the preceding claims, **characterised in that** the sonotrode (2) projects axially from the distal end of the first tube (10) by a predetermined lengthwise portion.

6. Ultrasonic disintegrator according to one of the preceding claims, **characterised in that** the sonotrode (2) and the first tube (8) are axially displaceable relative to each other.

7. Ultrasonic disintegrator according to one of the preceding claims, **characterised by** a second channel (26) which extends outside and alongside the sonotrode (2).

8. Ultrasonic disintegrator according to one of the preceding claims **characterised in that** the first channel (8; 24) and/or the second channel (26) is arranged at a predetermined location at the periphery of the sonotrode (2) and extends parallel to the sonotrode (2).

9. Ultrasonic disintegrator according to claim 7, **characterised in that** the second channel (26) is arranged at a predetermined location on the outside periphery of the first tube (10) and extends parallel to the sonotrode (2).

10. Ultrasonic disintegrator according to claim 7, **characterised in that** arranged concentrically around the first tube (10) a second tube (30) is arranged which forms the second channel (26) with an annular cross-section, which extends in the longitudinal direction of the sonotrode (2).

11. Ultrasonic disintegrator according to one of the preceding claims, **characterised in that** the proximal end of the sonotrode (2), the amplitude transformer (12) and the ultrasonic transducer (6) are accommodated in a housing (14), and **in that** the first channel (8) is guided through the housing (14).

12. Ultrasonic disintegrator according to one of the preceding claims, **characterised in that** the first or the second tube (10, 30) comprise a electrical conductor which can be connected to an external electrical HF-voltage source.

13. Ultrasonic disintegrator according to one of the preceding claims 1 to 11,
**characterised in that** the first and/or the second tube (10, 30) comprises an electrical insulator and the second electrode surface is a metal coating on the first or second tube (10, 30).

14. Ultrasonic disintegrator according to one of the preceding claims, **characterised in that** the sonotrode (2) is coated at least in sections by a dielectric insulating layer.

15. Ultrasonic disintegrator according to claim 12, **characterised in that** the first tube (10) and/or the second tube (30) on the inside surface are provided with a dielectric insulating layer.

16. Ultrasonic disintegrator according to one of the preceding claims, **characterised in that** the sonotrode (2) and the first tube (10) and possibly the second tube (30) are made from an elastic material.

17. Ultrasonic disintegrator according to one of the preceding claims, **characterised in that** the sonotrode (2) and the first tube (10) and possibly the second tube (30) can be secured to the instrument so that they can be replaced.

18. Ultrasonic disintegrator according to one of claims 1 to 17, **characterised in that** the wall of the first tube (10) is partially broken away at the distal end of the tube.

19. Ultrasonic disintegrator according to claim 18, **characterised in that** a peripheral portion is broken away from the wall of the first tube at the distal end of the tube.

20. Ultrasonic disintegrator according to claim 18 or 19, **characterised in that** the first axial tube (10) or the sonotrode (2) are axially displaceable by spring preloading.

21. Ultrasonic disintegrator according to claim 20, **characterised in that** the distal end (60) of the first tube (10), whose wall is broken away portion-wise at the periphery is displaceable axially under spring biasing force relative to a proximally adjoining tube portion (10a).

## Revendications

1. Désintégrateur à ultrasons, apte à être manoeuvré manuellement, destiné à l'ablation de tissu humain ou animal, comportant :
- une sonotrode (2) destinée à transmettre dans le tissu, au niveau de son extrémité distale (4), des ondes ultrasonores, la sonotrode (2) étant entourée par un premier tube (10), qui forme un premier canal (8) entre ledit tube et la sonotrode (2) et la sonotrode (2) étant réalisée dans un matériau électroconducteur, et le premier tube (10) comportant une deuxième surface d'électrode,
- un transducteur d'ultrasons (6), destiné à être raccordé à l'extrémité proximale de la sonotrode (2) par l'intermédiaire d'un convertisseur d'amplitude (12), et
- un premier canal (8), qui s'étend le long de la sonotrode (2), pour le rinçage ou l'aspiration du tissu désintégré et/ou enlevé
**caractérisé en ce que** l'extrémité distale de la sonotrode (2) comporte une surface pleine, qui est destinée à transmettre des ondes ultrasonores sur le tissu et qui ne comporte pas de petits évidements, et la sonotrode (2) dans la zone de son extrémité distale, y compris dans la surface pleine, forme une première surface d'électrode, et **en ce que** la première et la deuxième surface d'électrode peuvent être raccordées à une source de tension électrique à haute fréquence externe.

2. Désintégrateur à ultrasons selon la revendication 1, **caractérisé en ce que** la surface pleine au niveau de l'extrémité distale de la sonotrode (2) coïncide avec la face frontale distale (4) de la sonotrode (2).

3. Désintégrateur à ultrasons selon la revendication 1 ou 2, **caractérisé en ce que** la sonotrode (2) a une section pleine uniforme et une longueur qui correspond sensiblement à n x lambda/2, lambda étant la longueur d'onde de l'onde ultrasonore générée et n = 1, 2, 3, ....

4. Désintégrateur à ultrasons selon la revendication 1, **caractérisé en ce que** la sonotrode (2) est agencée au centre dans le premier tube (10).

5. Désintégrateur à ultrasons selon l'une quelconque des revendications précédentes, **caractérisé en ce que** la sonotrode (2) s'avance en saillie dans le sens axial hors de l'extrémité distale du premier tube (10) sur une longueur prédéfinie.

6. Désintégrateur à ultrasons selon l'une quelconque des revendications précédentes, **caractérisé en ce que** la sonotrode (2) et le premier tube (10) peuvent être déplacés dans le sens axial l'un par rapport à l'autre.

7. Désintégrateur à ultrasons selon l'une quelconque des revendications précédentes, **caractérisé par** un deuxième canal (26) qui s'étend à l'extérieur et le long de la sonotrode (2).

8. Désintégrateur à ultrasons selon l'une quelconque des revendications précédentes, **caractérisé en ce que** le premier canal (8 ; 24) et/ou le deuxième canal (26) sont agencés à un emplacement prédéfini sur le pourtour de la sonotrode (2) et sont parallèles à la sonotrode (2).

9. Désintégrateur à ultrasons selon la revendication 7, **caractérisé en ce que** le deuxième canal (26) est agencé à un emplacement prédéfini sur le pourtour extérieur du premier tube (10) et est parallèle à la sonotrode (2).

10. Désintégrateur à ultrasons selon la revendication 7, **caractérisé en ce qu'**un deuxième tube (30) est agencé concentriquement autour du premier tube (10) et forme le deuxième canal (26) à section annulaire, qui s'étend dans le sens longitudinal de la sonotrode (2).

11. Désintégrateur à ultrasons selon l'une quelconque des revendications précédentes, **caractérisé en ce que** l'extrémité proximale de la sonotrode (2), le convertisseur d'amplitude (12) et le transducteur d'ultrasons (6) sont logés dans un boîtier (14) et **en ce que** le premier canal (8) est guidé à travers le boîtier (14).

12. Désintégrateur à ultrasons selon l'une quelconque des revendications précédentes, **caractérisé en ce que** le premier et le deuxième tube (10, 30) sont formés par un conducteur électrique qui peut être raccordé à une source de tension électrique à haute fréquence externe.

13. Désintégrateur à ultrasons selon l'une quelconque des revendications 1 à 11, **caractérisé en ce que** le premier et/ou le deuxième tube (10, 30) sont réalisés dans un isolant électrique et la deuxième surface d'électrode est un revêtement métallique sur le premier ou le deuxième tube (10, 30).

14. Désintégrateur à ultrasons selon l'une quelconque des revendications précédentes, **caractérisé en ce que** la sonotrode (2) est revêtue au moins par zones par une couche isolante diélectrique.

15. Désintégrateur à ultrasons selon la revendication 12, **caractérisé en ce que** le premier tube (10) et/ou le deuxième tube (30) sont revêtus sur leur face intérieure d'une couche isolante diélectrique.

16. Désintégrateur à ultrasons selon l'une quelconque des revendications précédentes, **caractérisé en ce que** la sonotrode (2) et le premier tube (10), ainsi que, le cas échéant, le deuxième tube (30) sont réalisés dans un matériau élastique.

17. Désintégrateur à ultrasons selon l'une quelconque des revendications précédentes, **caractérisé en ce que** la sonotrode (2) et le premier tube (10), ainsi que, le cas échéant, le deuxième tube (30) peuvent être fixés de manière permutable sur l'instrument.

18. Désintégrateur à ultrasons selon l'une quelconque des revendications 1 à 7, **caractérisé en ce que** la paroi du premier tube (10) est partiellement rompue au niveau de l'extrémité distale du tube.

19. Désintégrateur à ultrasons selon la revendication 18, **caractérisé en ce qu'**une partie périphérique est rompue dans la paroi du premier tube au niveau de l'extrémité distale.

20. Désintégrateur à ultrasons selon la revendication 18 ou 19, **caractérisé en ce que** le premier tube (10) axial ou la sonotrode (2) peuvent être déplacés dans le sens axial sous l'effet d'une précontrainte par ressort.

21. Désintégrateur à ultrasons selon la revendication 20, **caractérisé en ce que** l'extrémité distale (60) du premier tube (10), dont la paroi est rompue sur une partie de la périphérie, peut être déplacée, dans le sens axial sous l'effet d'une précontrainte par ressort, contre une partie du tube (10a) adjacente en proximal.
